# EUROPEAN PATENT SPECIFICATION

(11) **EP 1 863 499 B1**
(45) Date of publication and mention of the grant of the patent: **19.08.2009**
(21) Application number: 06734212.1
(22) Date of filing: 01.02.2006
(51) Int. Cl.: A61K 31/7032, A61P 15/02, A61K 31/00

(54) **METHOD FOR INHIBITING AND/OR TREATING VAGINAL INFECTION**
VERFAHREN ZUR VORBEUGUNG UND/ODER BEHANDLUNG VAGINALER INFEKTIONEN
METHODE PERMETTANT D'INHIBER ET/OU DE TRAITER UNE INFECTION VAGINALE

(30) Priority: 30.03.2005 US 94496
(43) Date of publication of application: 12.12.2007
(73) Proprietor: KIMBERLY-CLARK WORLDWIDE, INC., Neenah, WI 54956 (US)
(72) Inventor: HUANG, Lei, Duluth, GA 30097 (US); YANG, Shu-ping, Alpharetta, GA 30005 (US)
(74) Representative: Leidescher, Thomas
(86) International application number: PCT/US2006/003681
(87) International publication number: WO 2006/107386

(56) References cited:
- US-A- 3 812 250
- LIVENGOOD C H III ET AL: "RESOLUTION OF RESISTANT VAGINAL TRICHOMONIASIS ASSOCIATED WITH THE USE OF INTRAVAGINAL NONOXYNOL-9" OBSTETRICS AND GYNECOLOGY, vol. 78, no. 5 PART 2, 1991, pages 954-956, XP009066511 ISSN: 0029-7844
- JONES B M ET AL: "THE SUSCEPTIBILITY OF ORGANISMS ASSOCIATED WITH BACTERIAL VAGINOSIS TO SPERMICIDAL COMPOUNDS IN-VITRO" GENITOURINARY MEDICINE, vol. 67, no. 6, 1991, pages 475-477, XP009066512 ISSN: 0266-4348
- FU Y ET AL: "CLONING AND CHARACTERIZATION OF A GENE (LIP1) WHICH ENCODES A LIPASE FROM THE PATHOGENIC YEAST CANDIDA ALBICANS" MICROBIOLOGY, SOCIETY FOR GENERAL MICROBIOLOGY, READING, GB, vol. 143, no. 2, 1997, pages 331-340, XP001037888 ISSN: 1350-0872

## Description

### Background of the Invention

The female vagina is naturally colonized by a variety of bacteria, yeast, and microorganisms. For example, a normal vagina generally contains more than about 10⁴ lactobacilli per milliliter of vaginal material. Under normal conditions, the vagina flora provides a mildly acidic environment that helps guard against the invasion of pathogenic microbes. Unfortunately, this vaginal balance may be easily upset by a variety of external factors that ultimately lead to vaginal infection. Vaginal infection is a clinical syndrome and exists in three primary forms, i.e., bacterial vaginosis, candidal vaginitis ("yeast"), and trichomonas vaginitis ("trich").

Bacterial vaginosis, for example, is a polymicrobial vaginal infection believed to be caused by an increase in the number of anaerobic organisms with a concomitant decrease in lactobacilli in the vagina. The decrease in the number of lactobacilli in the vagina has the dual effect of decreasing competition for nutrients and decreasing the amount of lactic acid present (i.e., increasing the pH). This allows for the multiplication of opportunistic pathogens in the vagina, whose growth is normally suppressed by the lactobacilli and the acidic pH of the vagina. The principal pathogen associated with bacterial vaginosis is believed to be *Gardnerella vaginalis*. Symptoms of bacterial vaginosis generally include an unpleasant smell, an elevated vaginal pH greater than about 5.0, a thin homogeneous discharge, and the presence of *Gardnerella* clue cells (i.e., vaginal epithelial cells coated with small Gram-variable rods). Current treatment regimens for bacterial infection of the vagina involve the use of various broad spectrum antibiotics, such as metronidazole. However, antibiotics are often undesirable because they may kill a broad range of the normal bacterial flora in the vagina, including the beneficial lactobacilli. This may cause secondary complications, because the lactobacilli keep various opportunistic pathogens in the vagina in check. The treatment may then necessitate a further treatment regimen, such as the ingestion of cultured dairy products to replace the lactobacilli in the body, as well as treatment by antifungal agents. Moreover, a rise in the level of anaerobes due to a lack of lactobacilli could further complicate the infection. Additionally, antibiotics, when used frequently within the vagina, may cause systemic toxicity through absorption from the vagina.

In addition, trichomonas vaginitis (or "trich") is one of the most common vaginal infections and is considered a sexually transmitted disease. Symptoms of trichomonas vaginitis include vulvar itching and odorous vaginal discharge. Trichomonas vaginitis is caused by *Trichomonas vaginalis*, a single-celled protozoan parasite not normally found in the flora of the genitourinary tract. *Trichomonas vaginalis* is a flagellate protozoa that is pear-shaped and about the size of a white blood cell. These motile cells have four flagellae and a single nucleus. Like bacterial vaginosis, this pathology is generally treated with metronidazole.

Further, the yeast *Candida albicans* causes the disease known as candidiasis (or "thrush"), as well as vulvitis (or "vulval" infection). *Candida albicans* is present in most humans as a harmless commensal organism. Problems arise, however, when a person experiences a loss of normal bacterial flora. In severely immune compromised patients, for example, *Candida albicans* infection may spread throughout the body and cause systemic infections. Candidiasis is usually treated with fluconazole, but this may have serious side affects and is not recommended for use during pregnancy.

One technique that has been developed for overcoming the problem with conventional treatments for vaginal infection is described in U.S. Patent Application Publication No. 2002/0114776 to Zaneveld, et al. The technique of Zaneveld, et al. involves the application of an effective amount polystyrene sulfonate into the vagina of a female. However, one problem with anionic compounds, such as polystyrene sulfonate, is that they are not generally biocompatible. That is, the anionic nature of such compounds is believed to irritate the skin or tissue of the use. In addition, such synthetic polymeric materials are also not readily biodegradable.

Livengood, C.H. III, et al., "Resolution of resistant vaginal Trichomoniasis associated with the use of intravaginal nonoxynol-9", Obstretics & Gynecology 78: 954-956 (1991) describes studies on treating Trichomonas infections with nonoxynol-9.

Jones, B.M., et al., "The susceptibility of organisms associated with bacterial vaginosis to spermicidal compounds, in vitro", Genitourinary Medicine 67: 475-477 (1991) describes studies on the activity of nonoxynol-9, nonoxynol-11, docusate sodium, benzalkonium chloride and Menfegol against Gardnerella vaginalis, Bacteroides and Mobiluncus organisms.

Fu, Y., et al., "Cloning and characterization of a gene (LIP1) which encodes a lipase from the pathogenic yeast Candida albicans", Microbiology 143:,331-340 (1997) discloses the cloning and characterization of a lipase from Candida albicans.

US-A-3,812,250 discloses a vaginal suppository comprising gentian violet, boric acid, gelatine, a non-ionic surfactant of the octylphenoxyethanol type, and water.

As such, a need currently exists for a biocompatible and biodegradable treatment composition for inhibiting and/or treating vaginal infection.

### Summary of the Invention

Subject matter of the present application is a use as defined in claim 1. The dependent claims relate to preferred embodiments thereof.

According to the present invention, there is disclosed the use of a saccharide-based non-ionic surfactant for the preparation of a treatment composition for inhibiting and/or treating infection in a vagina by exposing one or more microbes to said treatment composition, the microbes being selected from the group consisting of *Gardnerella, Candida,* and *Trichomonas,* said treatment composition comprising an effective amount of said saccharide-based non-ionic surfactant, wherein said saccharide-based non-ionic surfactant is an alkyl glycoside having the following formula:

(Z)ₙ-O-R

wherein,
Z is a saccharide residue;
n is from about 1 to about 1000; and
R is an alkyl group having 8 to 30 carbon atoms.

Other features and aspects of the present invention are discussed in greater detail below.

### Detailed Description of Representative Embodiments

Reference now will be made in detail to various embodiments of the invention, one or more examples of which are set forth below. Each example is provided by way of explanation of the invention, not limitation of the invention. In fact, it will be apparent to those skilled in the art that various modifications and variations may be made in the present invention without departing from the scope or spirit of the invention. For instance, features illustrated or described as part of one embodiment, may be used on another embodiment to yield a still further embodiment. Thus, it is intended that the present invention covers such modifications and variations as come within the scope of the appended claims and their equivalents.

Generally speaking, the present invention is directed to the use of a saccharide-based nonionic for the preparation of a treatment composition for inhibiting and/or treating infection in a vagina. surfactant. One benefit of such saccharide-based nonionic surfactants is that they are generally biocompatible and biodegradable. For example, nonionic surfactants are generally more compatible and less irritating to mammalian skin and tissue than ionic surfactants (e.g., amphoteric, cationic, or anionic surfactants). In addition to the benefits imparted by the nonionic nature of the surfactants of the present invention, the particular constituents of the surfactant also enhance biocompatibility and biodegradability. For instance, the hydrophilic group of saccharide-based surfactants is a residue of a monosaccharide, disaccharide (2 monosaccharide residues), trisaccharide (3 monosaccharide residues). oligosaccharide (4 to 20 monosaccharide residues), and/or polysaccharide (e.g.. more than 20 monosaccharide residues). Specific examples of such saccharide residues include, for instance, residues of glucose, fructose, mannose, xylose, lactose, maltose, maltotriose, galactose, dextrose, sucrose, leucrose, etc. Due to their natural derivation, such saccharide residues are generally thought to be compatible with biological materials, such as skin and tissue. In addition, the constituents of the saccharide-based surfactants are also formed from biodegradable and renewable resources, such as sugars and fatty alcohols obtained from natural oils (e.g. palm kernel oil, coconut oil, etc.).

The saccharide-based nonionic surfactants of the present invention are also capable of inhibiting and/or treating vaginal infection. Specifically, the saccharide-based nonionic surfactants possess a lipophilic (or hydrophobic) group and a hydrophilic group. The hydrophobic group is a long chain alkyl group. Without intending to be limited by theory, it is believed that the hydrophobic group of the nonionic surfactant may disrupt the lipid in the cell membrane of certain bacteria and parasites to an extent sufficient to kill or inhibit growth. To facilitate a balance between biocompatibility and antimicrobial effectiveness, it is sometimes desired that the hydrophilic/lipophilic balance ("HLB") of the nonionic surfactants be kept within a certain range. The HLS index is well known in the art and is a scale that measures the balance between the hydrophilic and lipophilic solution tendencies of a compound. The HLB scale ranges from 1 to approximately 50. with the lower numbers representing highly lipophilic tendencies and the higher numbers representing highly hydrophilic tendencies. Typically, the HLB of the surfactants of the present invention is from about 8 to about 18, in some embodiments from about 10 to about 16 and in some embodiments, from about 12 to about 14.

The saccharide-based nonionic surfactant is placed into contact with a female vagina in an effective amount to achieve the desired level of inhibition and/or treatment. An "effective amount" is an amount sufficient to inactivate, but not necessarily kill, pathogenic microorganisms responsible for vaginitis or bacterial vaginosis on contact. In fact, although not required, it may be desired to use a surfactant concentration that does not significantly affect or inhibit the growth characteristics of the normal vaginal flora or otherwise significantly irritate the vaginal tissue when used at inhibitory, noncytotoxic, or clinical concentrations. For example, the saccharide-based nonionic surfactant is desirably employed at a concentration of about 1 milligram per milliliter (mg/ml) to about 100 mg/ml, in some embodiments from about 2 to about 40 mg/ml, and in some embodiments, from about 5 mg/ml to about 20 mg/ml, based on the total weight of inert and active ingredients.

When administered in accordance with the present invention, the saccharide-based nonionic surfactant may provide selective inhibition for the growth of *Gardnerella, Candida,* and/or *Trichomonas* microbes. Specific species of such microbes that are inhibited by the saccharide-based nonionic surfactant include *Gardnerella vaginalis, Candida albicans,* and/or *Trichomonas vaginalis.* For example, it has been determined that treatment with the saccharide-based nonionic surfactant may provide a log reduction for *Candida albicans, Gardnerella vaginalis*, and/or *Trichomonas vaginalis* of at least about 2. in some embodiments at least about 3, in some embodiments at least about 4, and in some embodiments, at least about 5 (e.g., about 6). Log reduction, for example, may be determined from the % population killed by the formulation according to the following correlations:

| % Reduction | Log Reduction |
|---|---|
| 90 | 1 |
| 99 | 2 |
| 99.9 | 3 |
| 99.99 | 4 |
| 99.999 | 5 |
| 99.9999 | 6 |

The saccharide-based nonionic surfactant according to the present invention is an alkyl glycoside

(Z)ₙ-O-R

wherein,
Z is a saccharide residue;
n is from about 1 to about 1000; and
R is an alkyl group having 8 to 30 carbon atoms.

The "Z" saccharide residue of the alkyl glycoside typically has at least 3 carbon atoms, and in some embodiments, from about 3 to about 20 carbon atoms, and in some embodiments from about 5 to about 6 carbon atoms. The saccharide residue may, for instance, be a residue of glucose, fructose, maltose, maltotriose, lactose, galactose, mannose, dextrose, xylose, sucrose, leucrose, and so forth. The designation "n" represents the average number of saccharide residues in a particular sample of alkyl polyglycoside. For example, the alkyl glycoside may be a monosaccharide (n = 1), disaccharide (n = 2), trisaccharide (n = 3), oligosaccharide (n = 4 to 20), or polysaccharide (n > 20). In most embodiments, "n" is greater than about 2, in some embodiments from about 2 to about 6, and in some embodiments, from about 2 to about 4. The "alkyl group" of the alkyl glycosides is generally a linear alkyl group (i.e., a straight chain alcohol residue), which typically has an even number of carbon atoms. The alkyl glycosides desirably include alkyl groups having 8 to 20 carbon atoms, in some embodiments 8 to 14, and in some embodiments, 9 to 12. One particular example of a suitable alkyl glycoside is a mixture of alkyl glycoside molecules with alkyl chains having 8 to 10 carbon atoms.

The alkyl glycoside may include a single type of alkyl glycoside molecule or a mixture of different alkyl glycoside molecules. The different alkyl glycoside molecules may be isomeric and/or may be alkyl glycoside molecules with differing alkyl groups and/or saccharide residues. Alkyl glycoside isomers are alkyl polyglycosides which, although including the same alkyl ether residues, may vary with respect to the location of the alkyl ether residue in the alkyl glycoside, as well as isomers which differ with respect to the orientation of the functional groups about one or more chiral centers in the molecules. For example, an alkyl glycoside may include a mixture of molecules with saccharide residues that are mono-, di- or oligosaccharides derived from more than one 6 carbon saccharide residue and in which the mono-, di- or oligosaccharide has been etherified by reaction with a mixture of fatty alcohols of varying carbon chain length. When more than one saccharide residue is present on average per alkyl glycoside molecule (i.e., "n" is greater than 1), the individual saccharide subunits within the same molecule may be identical or different. When the individual subunits are not all identical, the order and distribution of subunits is typically random.

Alkyl glycosides may be produced using well-known techniques. Alkyl mono and polyglycosides are generally prepared by reacting a monosaccharide, or a compound hydrolyzable to a monosaccharide, with an alcohol such as a fatty alcohol in an acid medium. For example. U.S. Pat. Nos. 5,527,892 and 5,770,543. which are incorporated herein in their entirety by reference thereto for all purposes, describe alkyl glycosides and/or methods for their preparation. Commercially available examples of suitable alkyl glycosides include Glucopon^{™} 220, 225, 425, 600 and 625, all of which are available from Cognis Corp. of Cincinnati. Ohio. These products are mixtures of alkyl mono- and oligoglucopyranosides with alkyl groups based on fatty alcohols derived from coconut and/or palm kernel oil. Glucopon^{™} 220, 225 and 425 are examples of particularly suitable alkyl polyglycosides. Glucopon^{™} 220 is an alkyl polyglycoside that contains an average of 1.4 glucosyl residues per molecule and a mixture of 8 and 10 carbon alkyl groups (average carbons per alkyl chain-9.1). Glucopon^{™} 225 is a related alkyl polyglycoside with linear alkyl groups having 8 or 10 carbon atoms (average alkyl chain-9.1 carbon atoms) in the alkyl chain. Glucopon^{™} 425 includes a mixture of alkyl polyglycosides that individually include an alkyl group with 8, 10. 12, 14 or 16 carbon atoms (average alkyl chain-10.3 carbon atoms). Glucopon^{™} 600 includes a mixture of alkyl polyglycosides that individually include an alkyl group with 12, 14 or 16 carbon atoms (average alkyl chain 12.8 carbon atoms). Glucopon^{™} 625 includes a mixture of alkyl polyglycosides that individually include an alkyl group having 12. 14 or 18 carbon atoms (average alkyl chain 12.8 carbon atoms). Still other suitable alkyl glycosides are available from Dow Chemical Co. of Midland, Michigan under the Triton^{™} designation, e.g.. Triton^{™} CG-110 and BG-10.

The saccharide-based nonionic surfactant of the present invention may be prepared and applies in any suitable form. Desirably, the saccharide-based nonionic surfactant is formulated into a treatment composition to aid in topical application. The treatment composition may be in the form of a douche formulation, lotion, cream, jelly, liniment, ointment, salve, oil, foam, gel, film, wash, suppository, slow-releasing polymer, coating, etc. In actual use, the saccharide-based nonionic surfactant is applied into the vagina using any suitable technique (e.g., by hand, via gel or suppositories, or by using conventional tampon or syringe techniques).

To facilitate the incorporation of the saccharide-based nonionic surfactant into a treatment composition, a carrier is generally utilized. Although a variety of compounds may be employed for this purpose, it is usually desired that water act as the carrier to optimize biocompatibility. Other possible carriers include nonaqueous solvents, including glycols, such as propylene glycol, butylene glycol, triethylene glycol, hexylene glycol, polyethylene glycols, ethoxydiglycol, and dipropyleneglycol; alcohols, such as ethanol, n-propanol, and isopropanol; triglycerides; ethyl acetate; acetone; triacetin; and combinations thereof. Typically, the carrier is present in an amount greater than about 75 wt.%, in some embodiments greater than about 90 wt.%, and in some embodiments, from about 90 wt.% to about 99.99 wt.% of the treatment composition.

Whatever its form, the treatment composition may optionally include additional ingredients to impart various benefits. For instance, besides the saccharide-based nonionic surfactant, the treatment composition may also employ other surfactants to enhance the wettability of the composition on a substrate, to help emulsify or dissolve other ingredients, to increase viscosity, etc. If desired, the additional surfactants may include nonionic surfactants, such as ethoxylated alkylphenols, ethoxylated and propoxylated fatty alcohols, ethylene oxide-propylene oxide block copolymers, ethoxylated esters of fatty (C₈ -C₁₈) acids, condensation products of ethylene oxide with long chain amines or amides, condensation products of ethylene oxide with alcohols, and mixtures thereof. Various specific examples of suitable nonionic surfactants include, but are not limited to, methyl gluceth-10, PEG-20 methyl glucose distearate, PEG-20 methyl glucose sesquistearate, C₁₁₋₁₅ pareth-20, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene-10 oleyl ether, polyoxyethylene-20 oleyl ether, an ethoxylated nonylphenol, ethoxylated octylphenol, ethoxylated dodecylphenol, or ethoxylated fatty (C₆ -C₂₂) alcohol, including 3 to 20 ethylene oxide moieties, polyoxyethylene-20 isohexadecyl ether, polyoxyethylene-23 glycerol laurate, polyoxy-ethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxy-ethylene-6 tridecyl ether, laureth-2, laureth-3, laureth-4, PEG-3 castor oil, PEG 600 dioleate, PEG 400 dioleate, and mixtures thereof.

In some cases, ionic surfactants (i.e., anionic, cationic, or amphoteric surfactants) may also be employed in the treatment composition. For instance, one class of amphoteric surfactants that may be used are derivatives of secondary and tertiary amines having aliphatic radicals that are straight chain or branched, wherein one of the aliphatic substituents contains from about 8 to 18 carbon atoms and at least one of the aliphatic substituents contains an anionic water-solubilizing group, such as a carboxy, sulfonate, or sulfate group. Some examples of amphoteric surfactants include, but are not limited to, sodium 3-(dodecylamino)propionate, sodium 3-(dodecylamino)-propane-1-sulfonate, sodium 2-(dodecylamino)ethyl sulfate, sodium 2-(dimethylamino)octadecanoate, disodium 3-(N-carboxymethyl-dodecylamino)propane-1-sulfonate, disodium octadecyliminodiacetate, sodium 1-carboxymethyl-2-undecylimidazole, and sodium N, N-bis(2-hydroxyethyl)-2-sulfato-3-dodecoxypropylamine. Additional classes of amphoteric surfactants include phosphobetaines and the phosphitaines. For instance, some examples of such amphoteric surfactants include, but are not limited to, sodium coconut N-methyl taurate, sodium oleyl N-methyl taurate, sodium tall oil acid N-methyl taurate, sodium palmitoyl N-methyl taurate, cocodimethylcarboxymethylbetaine, lauryldimethylcarboxymethylbetaine, lauryldimethylcarboxyethylbetaine, cetyldimethylcarboxymethylbetaine, lauryl-bis-(2-hydroxyethyl)carboxymethylbetaine, oleyldimethylgammacarboxypropylbetaine, lauryl-bis-(2-hydroxypropyl)-carboxyethylbetaine, cocoamidodimethylpropylsultaine, stearylamidodimethylpropylsultaine, laurylamido-bis-(2-hydroxyethyl)propylsultaine, di-sodium oleamide PEG-2 sulfosuccinate, TEA oleamido PEG-2 sulfosuccinate, disodium oleamide MEA sulfosuccinate, disodium oleamide MIPA sulfosuccinate, disodium ricinoleamide MEA sulfosuccinate, disodium undecylenamide MEA sulfosuccinate, disodium wheat germamido MEA sulfosuccinate, disodium wheat germamido PEG-2 sulfosuccinate, disodium isostearamideo MEA sulfosuccinate, cocoamphoglycinate, cocoamphocarboxyglycinate, lauroamphoglycinate, lauroamphocarboxyglycinate, capryloamphocarboxyglycinate, cocoamphopropionate, cocoamphocarboxypropionate, lauroamphocarboxypropionate, capryloamphocarboxypropionate, dihydroxyethyl tallow glycinate, cocoamido disodium 3-hydroxypropyl phosphobetaine, lauric myristic amido disodium 3-hydroxypropyl phosphobetaine, lauric myristic amido glyceryl phosphobetaine, lauric myristic amido carboxy disodium 3-hydroxypropyl phosphobetaine, cocoamido propyl monosodium phosphitaine, lauric myristic amido propyl monosodium phosphitaine, and mixtures thereof.

Moreover, exemplary anionic surfactants include alkyl sulfates, alkyl ether sulfates, alkyl ether sulfonates, sulfate esters of an alkylphenoxy polyoxyethylene ethanol, α-olefin sulfonates, β-alkoxy alkane sulfonates, alkylauryl sulfonates, alkyl monoglyceride sulfates, alkyl monoglyceride sulfonates, alkyl carbonates, alkyl ether carboxylates, fatty acids, sulfosuccinates, sarcosinates, octoxynol or nonoxynol phosphates, taurates, fatty taurides, fatty acid amide polyoxyethylene sulfates, isethionates, or mixtures thereof. Particular examples of anionic surfactants include, but are not limited to, C₈-C₁₈ alkyl sulfates, C₈-C₁₈ fatty acid salts, C₈-C₁₈ alkyl ether sulfates having one or two moles of ethoxylation, C₈-C₁₈ alkamine oxides, C₈-C₁₈ alkoyl sarcosinates, C₈-C₁₈ sulfoacetates, C₈-C₁₈ sulfosuccinates, C₈-C₁₈ alkyl diphenyl oxide disulfonates, C₈-C₁₈ alkyl carbonates, C₈-C₁₈ alpha-olefin sulfonates, methyl ester sulfonates, and blends thereof. The C₈-C₁₈ alkyl group may be straight chain (e.g., lauryl) or branched (e.g., 2-ethylhexyl). The cation of the anionic surfactant may be an alkali metal (e.g., sodium or potassium), ammonium, C₁-C₄ alkylammonium (e.g., mono-, di-, tri-), or C₁-C₃ alkanolammonium (e.g., mono-, di-, tri). More specifically, such anionic surfactants may include, but are not limited to, lauryl sulfates, octyl sulfates, 2-ethylhexyl sulfates, lauramine oxide, decyl sulfates, tridecyl sulfates, cocoates, lauroyl sarcosinates, lauryl sulfosuccinates, linear C₁₀ diphenyl oxide disulfonates, lauryl sulfosuccinates, lauryl ether sulfates (1 and 2 moles ethylene oxide), myristyl sulfates, oleates, stearates, tallates, ricinoleates, cetyl sulfates, and similar surfactants.

When employed, it is generally desired that the additional surfactants be kept a concentration low enough so as not to affect the biocompatibility or antimicrobial effectiveness of the saccharide-based nonionic surfactant of the present invention. For example, ionic surfactants may sometimes have an adverse effect on biocompatibility. Thus, in most embodiments of the present invention, the additional surfactants comprise less than about 0.1 wt.%, and more desirably less than about 0.01 wt.% of the treatment composition.

Further, although the saccharide-based nonionic surfactant of the present invention is effective in inhibiting and/or treating vaginal infection, it may be desired in some cases to use other conventional antimicrobial agents. In one embodiment, for example, the antimicrobial agent is a quaternary ammonium compound, such as benzalkonium chloride (BZK) or other benzalkonium halides. Commercially available examples of such quaternary ammonium compounds are believed to include BARDAC® 2050 and BARDAC® 2080 (based on dialkyl(C₈ -C₁₀)dimethyl ammonium chloride); BARDAC® 2250 and BARDAC® 2280 (didecyl dimethyl ammonium chloride); BARDAC® LF and BARDAC® LF 80 (based on dioctyl dimethyl ammonium chloride); BARQUAT® MB-50 and BARQUAT® MB-80 (based on alkyl dimethyl benzyl ammonium chloride); BARQUAT® MX-50 and BARQUAT® MX-80 (based on alkyl dimethyl benzyl ammonium chloride); BARQUAT® OJ-50 and BARQUAT® OJ-80 (based on alkyl dimethyl benzyl ammonium chloride); BARQUAT® 4250, BARQUAT® 4280, BARQUAT® 4250Z, and BARQUAT® 4280Z (based on alkyl dimethyl benzyl ammonium chloride and/or alkyl dimethyl ethyl benzyl ammonium chloride); and BARQUAT® MS-100 (based on myristyl dimethyl benzyl ammonium chloride), which are available from Lonza, Inc., Fairlawn, N.J. Still other antimicrobial agents may also be utilized in the present invention. For instance, some suitable antimicrobial agents that may be utilized include, but are not limited to, halogenated diphenyl ethers like 2,4,4'-trichloro-2'-hydroxy-diphenyl ether (Triclosan® or TCS) or 2,2'-dihydroxy-5,5'-dibromo-diphenyl ether; phenolic compounds like phenoxyethanol, phenoxy propanol, phenoxyisopropanol, para-chloro-meta-xylenol (PCMX), etc.; bisphenolic compounds like 2,2'-methylene bis (4-chlorophenol), 2,2'-methylene bis (3,4,6-trichlorophenol), 2,2'-methylene bis (4-chloro-6-bromophenol), bis (2-hydroxy-3,5-dichlorophenyl) sulphide, and bis (2-hydroxy-5-chlorobenzyl)sulphide; halogenated carbanilides (e.g., 3,4,4'-trichlorocarbanilides (Triclocarban® or TCC); benzyl alcohols; chlorhexidine; chlorhexidine gluconate; and chlorhexidine hydrochloride. Still other antimicrobial agents are described in WO 96/06152; WO 96/06153; and U.S. Patent No. 6,201,695 to Beerse, et al., which are incorporated herein in their entirety by reference thereto for all purposes.

The amount of the additional antimicrobial agents utilized in the treatment composition may generally vary. It is normally desired that such antimicrobial agents be employed in a low enough amount so as not to adversely impact the biocompatibility of the treatment composition. Thus, in most embodiments of the present invention, the antimicrobial agent comprises less than about 0.1 wt.%, and more desirably less than about 0.01 wt.% of the treatment composition.

The treatment composition may also contain one or more preservatives. Although not required, preservatives may further inhibit the growth of microorganisms. Some suitable preservatives that may be used in the present invention include, but are not limited to, Kathon CG®, which is a mixture of methylchloroisothiazolinone and methylisothiazolinone available from Rohm & Haas; Mackstat H 66 (available from Mclntyre Group, Chicago, IL); DMDM hydantoin (e.g., "Glydant Plus", available from Lonza, Inc. of Fair Lawn, NJ); iodopropynyl butylcarbamate; benzoic esters (parabens), such as methylparaben, propylparaben, butylparaben, ethylparaben, isopropylparaben, isobutylparaben, benzylparaben, sodium methylparaben, and sodium propylparaben; 2-bromo-2-nitropropane-1,3-diol; benzoic acid; amidazolidinyl urea; diazolidinyl urea; and so forth. Other suitable preservatives include those sold by Sutton Labs, such as "Germall 115" (amidazolidinyl urea), "Germall II" (diazolidinyl urea), and "Germall Plus" (diazolidinyl urea and iodopropynyl butylcarbonate). When utilized, the amount of the preservative utilized in the treatment composition may generally vary depending on the relative amounts of the other components present within the composition. For example, in some embodiments, the preservative is present in the formulation in an amount between about 0.001 % to about 5% by weight, in some embodiments between about 0.001 to about 1% by weight, and in some embodiments, between about 0.1 % to about 0.15% by weight of the composition.

The pH of the treatment composition may be controlled to be within a range that is considered more biocompatible. For instance, it is typically desired that the pH be maintained at a mildly acidic level to correspond to normal vaginal conditions. For example, the pH may be less than about 7, in some embodiments from about 4 to about 6, and in one embodiment, about 5 (e.g., 4.7). If necessary, various pH modifiers may be utilized in the treatment composition to achieve the desired pH level. For instance, some examples of basic pH modifiers that may be used in the present invention include, but are not limited to, ammonia; mono-, di-, and tri-alkyl amines; mono-, di-, and tri-alkanolamines; alkali metal and alkaline earth metal hydroxides; alkali metal and alkaline earth metal silicates; and mixtures thereof. Specific examples of basic pH modifiers are ammonia; sodium, potassium, and lithium hydroxide; sodium, potassium, and lithium meta silicates; monoethanolamine; triethylamine; isopropanolamine; diethanolamine; and triethanolamine. Moreover, some examples of acidic pH modifiers that may be used in the present invention include, but are not limited to, mineral acids, sulfonic acids (e.g., 2-[N-morpholino] ethane sulfonic acid), carboxylic acids, and polymeric acids. Specific examples of suitable mineral acids are hydrochloric acid, nitric acid, phosphoric acid, and sulfuric acid. Specific examples of suitable carboxylic acids are citric acid, glycolic acid, lactic acid, maleic acid, malic acid, succinic acid, glutaric acid, benzoic acid, malonic acid, salicylic acid, gluconic acid, and mixtures thereof. Specific examples of suitable polymeric acids include straight-chain poly(acrylic) acid and its copolymers (e.g., maleic-acrylic, sulfonic-acrylic, and styrene-acrylic copolymers), cross-linked polyacrylic acids having a molecular weight of less than about 250,000, poly(methacrylic) acid, and naturally occurring polymeric acids such as carageenic acid, carboxymethyl cellulose, and alginic acid. When utilized, the pH modifier may be present in any effective amount needed to achieve the desired pH level. In some embodiments, the pH modifier is present in the composition in an amount between about 0.001 % to about 5% by weight, in some embodiments between about 0.001 to about 1% by weight, and in some embodiments, between about 0.1% to about 0.25% by weight of the treatment composition.

The treatment composition may also contain one or more drugs to impart a variety of different benefits. "Drugs" include any physiologically or pharmacologically active substance that produces a localized or a systemic effect in animals. The drugs that may be delivered include, but are not limited to, anti-inflammatory agents, analgesics, hormones, antihistamines, and so forth. Numerous such compounds are known to those of skill in the art and described, for example, in The Pharmacological Basis of Therapeutics, Hardman, Limbird, Goodman & Gilman, McGraw-Hill, New York, (1996), as well as U.S. Patent Nos. 6,419,913 to Niemiec, et al.; 6,562,363 to Mantelle, et al.; 6,593,292 to Rothbard, et al.; 6,567,693 to Allen, Jr.; and 6,645,181 to Lavi, et al., all of which are incorporated herein in their entirety by reference thereto for all purposes. One particularly useful class of drugs for vaginal applications is anti-inflammatory agents that reduce pain, swelling, stiffness, inflammation, etc. For example, nonsteroidal anti-inflammatory drugs (NSAIDs) may be utilized. Examples of NSAIDs include, but are not limited to, aspirin, ibuprofen, indomethacin, phenylbutazone, bromfenac, sulindac, nabumetone, ketorolac, mefenamic acid, and naproxen. Other suitable anti-inflammatory drugs are COX-2 inhibitors, such as celecoxib, meloxicam, rofecoxib, and flosulide. These drugs inhibit the production of the COX-2 (cyclooxygenase-2) enzyme induced by pro-inflammatory stimuli in migratory cells and inflamed tissue.

To better enhance the benefits to consumers, other optional ingredients may also be used. For instance, some classes of ingredients that may be used include, but are not limited to: antioxidants (product integrity); anti-reddening agents, such as aloe extract; astringents--cosmetic (induce a tightening or tingling sensation on skin); colorants (impart color to the product); deodorants (reduce or eliminate unpleasant odor and protect against the formation of malodor on body surfaces); fragrances (consumer appeal); opacifiers (reduce the clarity or transparent appearance of the product); skin conditioning agents; skin exfoliating agents (ingredients that increase the rate of skin cell turnover such as alpha hydroxy acids and beta hydroxyacids); skin protectants (a drug product which protects injured or exposed skin or mucous membrane surface from harmful or annoying stimuli); and thickeners (to increase the viscosity of the formulation).

The method of administering the saccharide-based nonionic surfactants of the present invention into the vagina is not critical so long as an effective amount of the surfactant is delivered. For example, the saccharide-based nonionic surfactant may be released into the vagina by hand, via gels or suppositories, or by using conventional tampon or syringe techniques. Generally, the treatment composition and/or method for delivering saccharide-based nonionic surfactant may allow it to remain within the vagina for an expended period of time (i.e., such as for more than about 2 hours after administration) to maximize effectiveness. It is normally desired that treatment begin as quickly as possible after the appearance of symptoms relating to vaginitis or bacterial vaginosis. The composition may be reapplied as needed, such as every 12 to 24 hours, until control is obtained. For prophylactic purposes, treatment about once a day may be sufficient. Of course, the frequency of application for either treatment or prophylactic purposes may vary with a number of factors, including, for example, the individual female and/or the severity of the infection.

The present invention may be better understood with reference to the following examples.

### EXAMPLE 1

The ability to inhibit pathogenic growth in accordance with the present invention was demonstrated. Specifically, an alkyl polyglycoside (APG) surfactant was obtained from Dow Chemical Co. of Midland, Michigan under the name "Triton™ CG-110." The Triton™ CG-110 surfactant was diluted to a concentration of 10 mg/ml using a 1X sterilized buffer solution (pH = 4.7) of (2-[N-morpholino] ethane sulfonic acid ("MES")). For testing, a suspension of *Candida albicans* (ATCC #10231) was prepared by diluting a 1 x 10⁸ colony forming unit (cfu) per milliliter (ml) stock of *Candida albicans* with a TSB solution to a concentration of 1 x 10⁵ cfu/ml. One milliliter of the suspension was applied to Dextrose Sabouraud agar plates, and allowed to grow at 35°C for 4 hours. 4-millimeter diameter wells were then punched in each agar plate, and 100 microliters of the sample was added to the wells. As a negative control, a 1X MES (pH = 4.7) buffer was employed. Further, as a positive control, a solution was employed that contained a benzyl quaternary ammonium chloride antimicrobial agent (diluted from BARDAC® 205M, from Lonza Inc., Fair Lawn, New Jersey) at a concentration of 10 mg/ml. The plates were incubated overnight at 35°C.

After 24 hours, the "zone of inhibition" for each sample was measured. The "zone of inhibition" is a circular zone formed around the agar plate in which the growth of the microorganism is inhibited. Absent treatment with an effective antimicrobial agent, the bacterial cells would normally produce an opaque lawn of growth. However, when growth is inhibited, a clear zone is observed. The diameter of this clear zone may thus be used as an indicator of antimicrobial effectiveness. In this particular example, the zone of inhibition for (1) the positive control was 10 millimeters, (2) the negative control was 0 millimeters, and (3) the test sample was 3 millimeters. Thus, the saccharide-based nonionic surfactant inhibited the growth of *Candida albicans.*

### EXAMPLE 2

The ability to inhibit pathogenic growth in accordance with the present invention was demonstrated. Specifically, the procedure of Example 1 was repeated for *Gardnerella vaginalis* (ATCC # 14018). The zone of inhibition for (1) the positive control (benzyl quaternary ammonium chloride, concentration of 5 mg/ml) was 4 millimeters, (2) the negative control was 0 millimeters, and (3) the test sample was 7 millimeters. Thus, not only did the saccharide-based nonionic surfactant inhibit the growth of *Gardnerella vaginalis,* but it also performed better than the conventional benzyl quaternary ammonium antimicrobial agent.

### EXAMPLE 3

The ability to inhibit pathogenic growth in accordance with the present invention was demonstrated. Specifically, an alkyl polyglycoside (APG) surfactant was obtained from Dow Chemical Co. of Midland, Michigan under the name "Triton™ CG-110." The Triton™ CG-110 surfactant was diluted to concentrations of 1 mg/ml and 5 mg/ml using a 1X sterilized MES buffer (pH = 4.7). 0.9 milliliters of the surfactant solutions were then added to culture tubes. For testing, a suspension of *Candida albicans* (ATCC #10231) was prepared by diluting a 1 x 10⁸ colony forming unit (cfu) per milliliter (ml) stock of *Candida albicans* with a TSB solution to a concentration of 1 x 10⁶ cfu/ml. 0.1 milliliters of the suspension was then added to each culture tube, and allowed to grow at 35°C for 30 minutes. The samples in the culture tubes were then diluted at 1, 10 and 100 times, and 100 microliters of each dilution was plated onto agar plates with WASP (Whitely Automatic Spiral Plate) spiral plating equipment from Don Whitely Scientific, Ltd. The plates were incubated overnight at 35°C, and the colonies were counted on each plate by either ProtoCol® from Synbiosis of Frederick, MD or by hand count. As a negative control, a 1 X MES (pH = 4.7) buffer was also employed in the same manner described above. Duplicate tests were conducted for each compound and control.

The results are set forth below in Table 1.

**Table 1: Candida albicans Reduction Results**

| Sample | Average count per ml | % Reduction |
|---|---|---|
| 5 mg/ml APG | 0.00 X 10⁰ | 100.0% |
| 1 mg/ml APG | 2.00 X 10³ | 99.8% |
| Control | 1.32 X 10⁶ | 0.0% |

As indicated, good inhibition for *Candida albicans* was achieved even for the relatively low concentration of 1 mg/ml APG.

### EXAMPLE 4

The ability to inhibit pathogenic growth in accordance with the present invention was demonstrated. Initially, sterile LYI-S-2 media (AYCC medium 2152) was provided and adjusted to a pH of 6.0 using 1 N HCl. Thereafter, an alkyl polyglycoside (APG) surfactant was diluted into the LYI-S-2 media to concentrations of 5 mg/ml, 30 mg/ml, and 50 mg/ml. The surfactant was obtained from Dow Chemical Co. of Midland, Michigan under the name "Triton™ CG-110." 0.9 milliliters of the surfactant solutions were then added to screw-capped test tubes. For testing, a suspension of *Trichomonas vaginalis* (ATCC #30001) was prepared by diluting a 2.16 x 10⁶ colony forming unit (cfu) per milliliter (ml) stock with a LYI-S-2 media to a concentration of 1 x 10⁶ cfu/ml. 0.1 milliliters of the suspension was then added to each test tube, and allowed to grow at 35°C for 24 hours on a 15° horizontal slant. As a negative control, a LYI-S-2 media was also employed in the same manner described above. The test was repeated four (4) times for the test and control samples.

The results are set forth below in Table 2.

**Table 2: Average count per ml of Trichomonas vaginalis**

| Sample | Run 1 | Run 2 | Run 3 | Run 4 | Mean | % Reduction |
|---|---|---|---|---|---|---|
| 5 mg/ml APG | 0 | 0 | 0 | 0 | 0 | 100.0 |
| 30 mg/ml APG | 0 | 0 | 0 | 0 | 0 | 100.0 |
| 50 mg/ml APG | 0 | 0 | 0 | 0 | 0 | 100.0 |
| Control | 1.516 x 10⁶ | 1.520 x 10⁶ | 1.460 x 10⁶ | 1.400 x 10⁶ | 1.474 x 10⁶ | 0.0 |

As indicated, good inhibition for *Trichomonas vaginalis* was achieved.

## Claims

1. Use of a saccharide-based non-ionic surfactant for the preparation of a treatment composition for inhibiting and/or treating infection in a vagina by exposing one or more microbes to said treatment composition, the microbes being selected from the group consisting of *Gardnerella, Candida,* and *Trichomonas,* said treatment composition comprising an effective amount of said saccharide-based non-ionic surfactant, wherein said saccharide-based non-ionic surfactant is an alkyl glycoside having the following formula:
(Z)ₙ-O-R
wherein,
Z is a saccharide residue;
n is from about 1 to about 1000; and
R is an alkyl group having 8 to 30 carbon atoms.

2. The use of claim 1, wherein the microbes are selected from the group consisting of *Gardnerella vaginalis, Candida albicans,* and *Trichomonas vaginalis.*

3. The use of claim 1 or 2, wherein Z is a residue of glucose, fructose, maltose, maltotriose, lactose, galactose, mannose, dextrose, xylose, sucrose, leucrose, or combinations thereof.

4. The use of any of the foregoing claims, wherein the saccharide residue has 5 to 6 carbon atoms.

5. The use of any of the foregoing claims, wherein n is from about 2 to about 6.

6. The use of any of the foregoing claims, wherein the alkyl group has 8 to 20 carbon atoms.

7. The use of any of the foregoing claims, wherein the saccharide-based non-ionic surfactant is present in the treatment composition at a concentration of from about to about 100 milligrams per milliliter.

8. The use of any of the foregoing claims, wherein the saccharide-based non-ionic surfactant is present in the treatment composition at a concentration of from about 2 to about 40 milligrams per milliliter.

9. The use of any of the foregoing claims, wherein the saccharide-based non-ionic surfactant is present in the treatment composition at a concentration of from about 5 to about 20 milligrams per milliliter.

10. The use of any of the foregoing claims, wherein the saccharide-based non-ionic surfactant has an HLB of from about 8 to about 18.

11. The use of any of the foregoing claims, wherein the saccharide-based non-ionic surfactant has an HLB of from about 10 to about 16.

12. The use of any of the foregoing claims, wherein the treatment composition further comprises a carrier in an amount of greater than about 75 wt.% of the composition, and preferably greater than about 90 wt.% of the composition.

13. The use of claim 12, wherein the carrier is water.

14. The use of any of the foregoing claims, wherein the treatment composition further comprises an additional surfactant, antimicrobial agent, preservative, pH modifier, anti-inflammatory agent, or combination thereof.

15. The use of any of the foregoing claims, wherein the pH of the treatment composition is from about 4 to about 6.

16. The use of any of the foregoing claims, wherein the treatment composition is for administration into the vagina of a female who has or is at risk for a vaginal infection.

## Patentansprüche

1. Verwendung eines nicht-ionischen oberflächenaktiven Mittels auf Basis von Saccharid zur Herstellung einer Behandlungszusammensetzung zum Inhibieren und/oder Behandeln von Infektion in einer Vagina, indem man eine oder mehrere Mikroben der Behandlungszusammensetzung aussetzt, wobei die Mikroben ausgewählt werden aus der Gruppe, bestehend aus *Gardnerella, Candida* und *Trichomonas,* die Behandlungszusammensetzung eine wirksame Menge des nicht-ionischen oberflächenaktiven Mittels auf Basis von Saccharid umfasst, und das nicht-ionische oberflächenaktive Mittel auf Basis von Saccharid ein Alkylglycosid mit der nachfolgenden Formel ist:
(Z)ₙ-G-R
wobei
Z ein Saccharidrest ist,
n von etwa 1 bis etwa 1000 beträgt, und
R eine Alkylgruppe mit 8 bis 30 Kohlenstoffatomen ist.

2. Verwendung nach Anspuch 1, wobei die Mikroben ausgewählt werden aus der Gruppe, bestehend aus *Gardnerella vaginalis, Candida albicans* und *Trichomonas vaginalis.*

3. Verwendung nach Anspruch 1 oder 2, wobei Z ein Rest von Glucose, Fructose, Maltose, Maltotriose, Lactose, Galactose, Mannose, Dextrose, Xylose, Sucrose, Leucrose oder Kombinationen davon ist.

4. Verwendung nach einem der vorhergehenden Ansprüche, wobei der Saccharidrest 5 bis 6 Kohlenstoffatome aufweist.

5. Verwendung nach einem der vorhergehenden Ansprüche, wobei n von etwa 2 bis etwa 6 beträgt.

6. Verwendung nach einem der vorhergehenden Ansprüche, wobei die Alkylgruppe 8 bis 20 Kohlenstoffatome aufweist.

7. Verwendung nach einem der vorhergehenden Ansprüche, wobei das nicht-ionische oberflächenaktive Mittel auf Basis von Saccharid in der Behandlungszusammensetzung in einer Konzentration von etwa 1 bis etwa 100 Milligramm pro Milliliter vorhanden ist.

8. Verwendung nach einem der vorhergehenden Ansprüche, wobei das nicht-ionische oberflächenaktive Mittel auf Basis von Saccharid in der Behandlungszusammensetzung in einer Konzentration von etwa 2 bis etwa 40 Milligramm pro Milliliter vorhanden ist.

9. Verwendung nach einem der vorhergehenden Ansprüche, wobei das nicht-ionische oberflächenaktive Mittel auf Basis von Saccharid in der Behandlungszusammensetzung in einer Konzentration von etwa 5 bis etwa 20 Milligramm pro Milliliter vorhanden ist.

10. Verwendung nach einem der vorhergehenden Ansprüche, wobei das nicht-ionische oberflächenaktive Mittel auf Basis von Saccharid einen HLB-Wert von etwa 8 bis etwa 18 aufweist.

11. Verwendung nach einem der vorhergehenden Ansprüche, wobei das nicht-ionische oberflächenaktive Mittel auf Basis von Saccharid einen HLB-Wert von etwa 10 bis etwa 16 aufweist.

12. Verwendung nach einem der vorhergehenden Ansprüche, wobei die Behandlungszusammensetzung des Weiteren einen Träger in einer Menge von mehr als etwa 75 Gew.-% der Zusammensetzung und bevorzugt von mehr als etwa 90 Gew.-% der Zusammensetzung umfasst.

13. Verwendung nach Anspruch 12, wobei der Träger Wasser ist.

14. Verwendung nach einem der vorhergehenden Ansprüche, wobei die Behandlungszusammensetzung des Weiteren ein zusätzliches oberflächenaktives Mittel, ein antimikrobielles Agens, ein Konservierungsmittel, einen pH-Modifikator, ein entzündungshemmendes Mittel oder eine Kombination davon umfasst.

15. Verwendung nach einem der vorhergehenden Ansprüche, wobei der pH-Wert der Behandlungszusammensetzung von etwa 4 bis etwa 6 beträgt.

16. Verwendung nach einem der vorhergehenden Ansprüche, wobei die Behandlungszusammensetzung vorgesehen ist zur Verabreichung in die Vagina einer weiblichen Person, welche eine vaginale Infektion hat oder gefährdet ist, eine vaginale Infektion zu bekommen.

## Revendications

1. Utilisation d'un tensioactif non ionique à base de saccharide pour la préparation d'une composition de traitement pour inhiber et/ou traiter une infection vaginale par exposition d'un ou plusieurs microbes à ladite composition de traitement, les microbes étant sélectionnés dans le groupe consistant en *Gardnerella, Candida* et *Trichomonas,* ladite composition de traitement comprenant une quantité efficace dudit tensioactif non ionique à base de saccharide, ledit tensioactif non ionique à base de saccharide étant un alkylglycoside ayant la formule suivante :
(Z)ₙ-O-R
dans laquelle :
Z est un résidu saccharide ;
n est compris entre environ 1 et environ 1000 ; et
R est un groupe alkyle ayant de 8 à 30 atomes de carbone.

2. Utilisation selon la revendication 1, dans laquelle les microbes sont sélectionnés dans le groupe consistant en *Gardnerella vaginalis, Candida albicans* et *Trichomonas vaginalis.*

3. Utilisation selon la revendication 1 ou 2, dans laquelle Z est un résidu glucose, fructose, maltose, maltotriose, lactose, galactose, mannose, dextrose, xylose, saccharose, leucrose, ou leurs combinaisons.

4. Utilisation selon l'une quelconque des revendications précédentes, dans laquelle le résidu saccharide a 5 ou 6 atomes de carbone.

5. Utilisation selon l'une quelconque des revendications précédentes, dans laquelle n est compris entre environ 2 et environ 6.

6. Utilisation selon l'une quelconque des revendications précédentes, dans laquelle le groupe alkyle a de 8 à 20 atomes de carbone.

7. Utilisation selon l'une quelconque des revendications précédentes, dans laquelle le tensioactif non ionique à base de saccharide est présent dans la composition de traitement à une concentration allant d'environ 1 à environ 100 milligrammes par millilitre.

8. Utilisation selon l'une quelconque des revendications précédentes, dans laquelle le tensioactif non ionique à base de saccharide est présent dans la composition de traitement à une concentration allant d'environ 2 à environ 40 milligrammes par millilitre.

9. Utilisation selon l'une quelconque des revendications précédentes, dans laquelle le tensioactif non ionique à base de saccharide est présent dans la composition de traitement à une concentration allant d'environ 5 à environ 20 milligrammes par millilitre.

10. Utilisation selon l'une quelconque des revendications précédentes, dans laquelle le tensioactif non ionique à base de saccharide a un HLB compris entre environ 8 et environ 18.

11. Utilisation selon l'une quelconque des revendications précédentes, dans laquelle le tensioactif non ionique à base de saccharide a un HLB compris entre environ 10 et environ 16.

12. Utilisation selon l'une quelconque des revendications précédentes, dans laquelle la composition de traitement renferme, en outre, un support en une quantité supérieure à environ 75 % en poids de la composition, et de préférence supérieure à environ 90 % en poids de la composition.

13. Utilisation selon la revendication 12, dans laquelle le support est l'eau.

14. Utilisation selon l'une quelconque des revendications précédentes, dans laquelle la composition de traitement comprend, en outre, un tensioactif supplémentaire, un agent antimicrobien, un conservateur, un agent modifiant le pH, un agent anti-inflammatoire, ou une combinaison de ceux-ci.

15. Utilisation selon l'une quelconque des revendications précédentes, dans laquelle le pH de la composition de traitement est compris entre environ 4 et environ 6.

16. Utilisation selon l'une quelconque des revendications précédentes, dans laquelle la composition de traitement est destinée à une administration vaginale chez un sujet de sexe féminin ayant, ou exposé au risque d'avoir, une infection vaginale.
